(19) Europäisches Patentamt
European Patent Office
Office européen des brevets

(11) **EP 3 837 801 B1**

(12) **EUROPEAN PATENT SPECIFICATION**

(45) Date of publication and mention
of the grant of the patent:
**07.06.2023 Bulletin 2023/23**

(51) International Patent Classification (IPC):
***H04L 9/06*** *(2006.01)*      ***H04L 9/08*** *(2006.01)*
***H04L 9/14*** *(2006.01)*

(21) Application number: **19755341.5**

(22) Date of filing: **13.08.2019**

(52) Cooperative Patent Classification (CPC):
**H04L 9/0643; H04L 9/0866; H04L 9/0869;
H04L 9/0894; H04L 9/14**

(86) International application number:
**PCT/EP2019/071743**

(87) International publication number:
**WO 2020/035503 (20.02.2020 Gazette 2020/08)**

(54) **COMPARING DATA RECORD ENTRIES**

VERGLEICHEN VON DATENSATZEINTRÄGEN

COMPARAISON D'ENTRÉES D'ENREGISTREMENT DE DONNÉES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **13.08.2018   GB 201813184**

(43) Date of publication of application:
**23.06.2021 Bulletin 2021/25**

(73) Proprietor: **Abducere Holdings Ltd
Hassocks
BN6 9NL (GB)**

(72) Inventors:
• **ARMOUR, Francis
Congleton, Cheshire CW12 2JZ (GB)**
• **WELLER, Andrew
Hassocks, West Sussex BN6 9NL (GB)**
• **ARBOLI LOPEZ, Servando Miguel
Harpenden, Hertfordshire AL5 3AR (GB)**

(74) Representative: **Townsend, Martyn
Page White & Farrer Limited
Bedford House
21A John Street
London WC1N 2BF (GB)**

(56) References cited:
**US-A1- 2008 137 840     US-A1- 2015 288 665**

EP 3 837 801 B1

## Description

Background

Many different organisations often hold information on the same data subject. For example:

- Different businesses may hold information on the same person, such as a customer or supplier,
- Different government departments or agencies might hold information about cars or other vehicles, and
- Different hospitals may hold information about the same patient or research topic, etc.

Currently, when organisations want to exchange between themselves information they hold about their shared, same subject, they can do so in two ways. One way is to share data on large pools of subjects which have similar characteristics, but where the data have been largely anonymised and cannot be linked with specific subjects. The other way is on an individual basis, where the data to be shared is specific to each subject.

Before two businesses can exchange data on shared persons, they first need to establish which of these they may have in common. For commercial, privacy or other reasons, they may need to accomplish this without divulging details of their entire customer bases to one another. In many cases, businesses utilize a third-party "match-making service", which compares their customers and establishes those which are common. Once a match has been established, the third party agency can then broker the sharing of certain details. Businesses commonly share customer data in this fashion.

Such agencies collect information from, for example, public records (e.g. the electoral register) and the various companies, organisations, etc., that a person has a relationship with. This can include government agencies, insurers, banks, credit card providers, utility suppliers, mobile phone companies, etc. The information held about a person might include, for example; an individual's name, current and previous addresses, number of current and previous bank accounts, payment information (e.g. missed or late payments), court judgements, etc.

However, an inherent weakness with the "match-making service" approach is that large volumes of personal data are shared between the organisations via the match-maker. Whilst this data might be transmitted securely using e.g. cryptographic techniques, it is usually stored and processed by third parties in the clear as plaintext. This creates an additional point of vulnerability for data loss or misuse. For example, there have been well-publicised examples of large agencies in the United Kingdom and USA suffering major data breaches in recent years, with millions of individual's personal information being stolen.

Moreover, with the introduction of the general data protection regulation (GDPR), if entities in the European Union want to exchange individual customer specific data, they can do so only if they have the express consent of that customer.

US20078/137840A1 discloses a computer implemented method for performing a privacy enhanced comparison of a plurality of data sets includes allocating a private encryption key to each of the data sets; performing an encryption operation for each of the data sets, the encryption operation comprising generating a commutatively encrypted data set of the respective data set, wherein the commutatively encrypted data sets are generated by successively applying a keyed commutative encryption function on the respective data set with the private encryption key of the respective data set itself and with the private encryption keys of the other data sets; and comparing the commutatively encrypted data sets.

US2015/288665A1 discloses that a secure linkage between databases allows records of an individual in a first database to be linked to records of the same individual in a second database without disclosing or providing personal information outside of either database or system responsible for controlling access to the respective databases. As such, records of individuals may be securely linked together without compromising privacy or security of the databases.

Summary

The inventors of the present invention have recognised that it would be advantageous for a party (e.g. a business or other organisation) to identify, at an individual level, data subjects (e.g. persons (or people) that they have in common with another party, without ever having to share any specific, identifying personal data.

For brevity and ease of explanation, the concepts of this invention will be set out using, as an example, the case of businesses wishing to share data about the same person, such as a customer. However, the concepts are equally applicable to any case where organisations wish to exchange information about the same, shared data subj ect.

According to a first aspect disclosed herein, there is provided a method of comparing data record entries of two or more parties, wherein each party maintains a data record comprising a plurality of entries, each entry representing a corresponding data subject and comprising one or more identifiers of that data subject. The method comprises operating computing equipment of a first one of the two or more parties to perform operations of: to each of one or more of the identifiers of the data subject corresponding to one of the entries in the data record of the first party, applying one or more common modification algorithms common to the two or more parties (common meaning that the same modification algorithms have been provided to each of the two or more parties), wherein each modification algorithm modifies the identifier to which it is applied to thereby generate a respective data value; and for each generated data value, inputting at least that data value to a common hash function common to the two or more parties in order to generate a respective key (common meaning that the same hash function has been provided to each of th two or more parties). Generating a respective key comprises, for each generated data value, inputting at least that data value to a first common hash function to generate a respective private key, and inputting one or more respective private keys generated by the first party to a second common hash function common to the two or more parties in order to generate a respective public key, wherein the one or more respective private keys input to the second common hash function are chosen based on a common entanglement algorithm common to the two or more parties, wherein the common entanglement algorithm prescribes which of the generated private keys and/or which parts of the generated private keys are input to the second common hash function. The method further comprise storing each generated key in a first key set and supplying the first key set to a comparison algorithm, wherein said storing comprises storing at least each generated public key in the first key set. The comparison algorithm is configured to determine whether said one of the data subjects corresponds to an entry in both the data record of the first party and a data record of a second, different one of the two or more parties by: (i) comparing one or more of the keys in the first key set with one or more keys in a second key set generated by the second party (the second party having generated the second key set based on the one or more common modification algorithms and common hash function as provided to the second party), and (ii) determining whether the compared key sets comprise one or more identical respective keys. Comparing the one or more keys comprises comparing one or more of the public keys in the first key set with one or more public keys in the second key set generated by the second party. Determining where the compared key sets comprise one or more identical respective keys comprises determining whether the compared key sets comprises one or more identical respective public keys. The method then further comprises receiving a result of the comparison algorithm, wherein the result indicates whether said one of the data subjects corresponds to an entry in both the data record of the first party and the data record of the second party.

Once a data subject has been confirmed as being known to two or more parties, those parties can share data at an individual level without ever having to betray a data subject's specific data which could be used to identify that data subject. For example, (non-identifying) data can be shared along with an identical key that is common to both parties. For instance, a first party (e.g. a bank) and a second party (e.g. an energy supplier) establish that they have a common person (e.g. customer) based on deriving an identical key from an identifier (e.g. name) of that person. Here, a person's very identity becomes the basis for their own unique key. The identical key is generated using a hash function and is therefore meaningless on its own. That is, it cannot be used to identify the person. The energy supplier may therefore send data to the bank along with the key. For example, the energy supplier may inform the bank that the person whose key is e.g. XHF31, has routinely paid their bills for three years without missing a payment. This way, both parties can share data anonymously.

In embodiments, the result may indicate a likelihood of whether said one of the data subjects corresponds to an entry in both the data record of the first party and the data record of the second party.

In embodiments, the comparison algorithm may be configured to determine whether said one of the data subjects corresponds to an entry in both the data record of the first party and the data record of the second party by determining whether the compared key sets comprise a number of identical keys greater than or equal to a threshold number.

In embodiments, the threshold number may be less than a number of keys in the first and/or second key sets.

In embodiments, the comparison algorithm may be an internal comparison algorithm performed by the computing equipment of the first party, wherein said supplying comprises receiving the second key set from the second party and supplying the receiving second key set to the internal comparison algorithm.

In embodiments, the comparison algorithm may be an external comparison algorithm performed by computing equipment of a third party match-making service external to the two or more parties, wherein the match-making service, and wherein said supplying comprises transmitting the first key set to said match-making service.

In embodiments, the method may comprise: receiving a common seed number common to the two or more parties for determining a common pseudorandom number (common meaning the same seed number has been provided to each of the two or more parties); and wherein said inputting comprises inputting the common seed number to at least one of the hash functions to generate the respective key.

In embodiments, the method may comprise updating the seed number used to determine the common pseudorandom number.

In embodiments, the method may comprise: for each of said data values, combining that data value with a same respective cryptographic salt common to the two or more parties (i.e. the same salt being provided to each of the two or more parties); wherein said inputting of at least the respective data value to the first hash function comprises inputting at least the respective data value with the combined cryptographic salt to the first hash function to generate the private key.

In embodiments, said inputting may comprise inputting at least the respective data value with the combined cryptographic salt and the determined pseudorandom number to at least one of the hash functions to generate the respective key.

In embodiments, the one or more modification algorithms may comprise one or more linguistic modification algorithms and/or one or more numerical modification algorithms.

In embodiments, the one or more keys may be a plurality of keys.

In embodiments, the one or more identifiers may comprise at least one of: a first name, a surname, a date of birth, a nationality, a city of birth, an address, a passport number, a national insurance number, a driving license number, a vehicle registration number, a company registration number, a contract number, an internet protocol address, and/or a biometric identifier.

In embodiments, the two or more parties may comprise at least one of: a credit reference agency, an insurance provider, a financial institution, a health service provider, an education provider, a judicial institution, a government institution, a utility service provider, a television service provider, and/or an internet service provider.

In embodiments, said applying may comprise applying a plurality of modification algorithms to the at least one of the identifiers of the data subject corresponding to one of the entries in the data record of the first party.

According to a second aspect disclosed herein, there is provided a method of comparing data record entries of two or more parties, wherein each party maintains a data record comprising a plurality of entries, each entry representing a corresponding data subject and comprising one or more identifiers of that data subject, wherein the method comprises operating computing equipment of a third-party match-making service other than the two or more parties to perform operations of: providing one or more common modification algorithms to each of the two or more parties, wherein each modification algorithm modifies the identifier to which it is applied to thereby generate a respective data value; and providing one or more common hash functions to each of the two or more parties, wherein each common hash function, when applied to the respective data value, generates a respective key, wherein said providing of the common hash functions comprises: providing a first common hash function to each of the two or more parties, wherein the first common hash function, when applied to the respective data value, generates a respective private key; and providing a second common hash function to each of the two or more parties, wherein the second common hash function, when applied to one or more of the private keys generated each party, generates a respective public key, and wherein the method further comprises: providing a common entanglement algorithm to each of the two or more parties, wherein the entanglement algorithm prescribes which of the generated private keys and/or which parts of the generated private keys are input to the second common hash function; receiving a first key set from a first one of the two or more parties, wherein the first key set comprises keys generated by the first party; receiving a second key set from a second one of the two or more parties, the first party and second party being different ones of the two or more parties, wherein the second key set comprises keys generated by the second party; publishing the first and/or second key set to one or more of the two or more parties; comparing one or more of the keys of the first key set with one or more of the keys of the second key set; determining whether said one of the data subjects corresponds to an entry in both the data record of the first party and the data record of the second party based on whether the compared key sets comprise one or more identical respective keys; and transmitting a result of said determination to the first and/or second party, wherein the result indicates whether said one of the data subjects corresponds to an entry in both the data record of the first party and the data record of the second party.

In embodiments, the result may indicate a likelihood of whether said one of the data subjects corresponds to an entry in both the data record of the first party and the data record of the second party.

In embodiments, the method may comprise: providing a common seed number to each of the two or more parties, wherein the common seed number is used by each of the two or more parties to determine a common pseudorandom number, wherein the common seed number is input to at least one of the hash functions to generate the respective key.

In embodiments, the method may comprise: providing an updated seed number to each of the two or more parties.

In embodiments, the method may comprise: providing, to each of the two or more parties, a same cryptographic salt for each respective data value, wherein at least the respective data value and the cryptographic salt are combined and input to the first hash function to generate the private key.

In embodiments, the method may comprise: providing, to each of the two or more parties, a same cryptographic salt for each respective private key, wherein at least the respective private key and the cryptographic salt are combined and input to the second hash function to generate the public key.

According to a third aspect disclosed herein, there is provided a computer program for comparing data record entries of two or more parties, wherein each party maintains a data record comprising a plurality of entries, each entry representing a corresponding data subject and comprising one or more identifiers of that data subject; wherein the computer program comprises instructions embodied on computer-readable storage and configured so as, when the program is executed by a computer, cause the computer to perform operations of: to each of one or more of the identifiers of the data subject corresponding to one of the entries in the data record of the first party, applying one or more common modification algorithms, wherein the one or more modification algorithms are common to the two or more parties, wherein each modification algorithm modifies the identifier to which it is applied to thereby generate a respective data value; for each generated data value, inputting at least that data value to a common hash function common to the two or more parties in order to generate a respective key, wherein said generating of the respective key comprises: for each generated data value, inputting at least that data value to a first common hash function to generate a respective private key; inputting one or more respective private keys generated by the first party to a second common hash function common to the two or more parties in order to generate a respective public key, wherein the one or more respective private keys input to the second common hash function are chosen based on a common entanglement algorithm common to the two or more parties, wherein the common entanglement algorithm prescribes which of the generated private keys and/or which parts of the generated private keys are input to the second common hash function; storing each generated key in a first key set, wherein said storing comprises storing at least each generated public key in the first key set; supplying the first key set to a comparison algorithm configured to determine whether said one of the data subjects corresponds to an entry in both the data record of the first party and a data record of a second, different one of the two or more parties by: (i) comparing one or more of the keys in the first key set with one or more keys in a second key set generated by the second party, and (ii) determining whether the compared key sets comprise one or more identical respective keys, wherein said comparing comprises comparing one or more of the public keys in the first key set with one or more public keys in the second key set generated by the second party, and wherein said determining comprises determining whether the compared key sets comprises one or more identical respective public keys; and receiving a result of the comparison algorithm, wherein the result indicates whether said one of the data subjects corresponds to an entry in both the data record of the first party and the data record of the second party.

According to a fourth aspect disclosed herein, there is provided a computer program for comparing data record entries of two or more parties, wherein each party maintains a data record comprising a plurality of entries, each entry representing a corresponding data subject and comprising one or more identifiers of that data subject; wherein the computer program comprises instructions embodied on computer-readable storage and configured so as,, when the program is executed by a computer of a third-party match-making service other than the two or more parties, cause the computer to perform operations of: providing one or more common modification algorithms to each of the two or more parties, wherein each modification algorithm modifies the identifier to which it is applied to thereby generate a respective data value; and providing one or more common hash functions to each of the two or more parties, wherein each common hash function, when applied to the respective data value, generates a respective key, wherein said providing of the common hash functions comprises: providing a first common hash function to each of the two or more parties, wherein the first common hash function, when applied to the respective data value, generates a respective private key; and providing a second common hash function to each of the two or more parties, wherein the second common hash function, when applied to one or more of the private keys generated each party, generates a respective public key, and wherein the operations further comprise: providing a common entanglement algorithm to each of the two or more parties, wherein the entanglement algorithm prescribes which of the generated private keys and/or which parts of the generated private keys are input to the second common hash function; receiving a first key set from a first one of the two or more parties, wherein the first key set comprises keys generated by the first party; receiving a second key set from a second one of the two or more parties, the first party and second party being different ones of the two or more parties, wherein the second key set comprises keys generated by the second party; publishing the first and/or second key set to one or more of the two or more parties; comparing one or more of the keys of the first key set with one or more of the keys of the second key set; determining whether said one of the data subjects corresponds to an entry in both the data record of the first party and the data record of the second party based on whether the compared key sets comprise one or more identical respective keys; and transmitting a result of said determination to the first and/or second party, wherein the result indicates whether said one of the data subjects corresponds to an entry in both the data record of the first party and the data record of the second party.

Brief Description of the Drawings

To assist understanding of the present disclosure and to show how embodiments may be put into effect, reference is made by way of example to the accompanying drawings in which:

Figure 1 shows schematically an example network of users and parties,

Figure 2 shows schematically an example of how keys generated by two or more parties may be generated and compared; and

Figure 3 shows schematically an example of how private and public hashes are generated.

Detailed Description

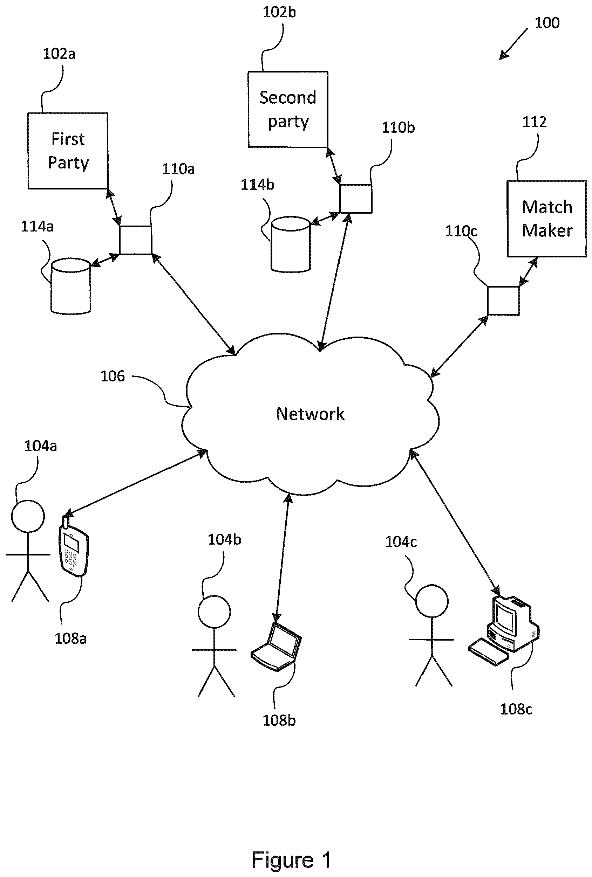

Figure 1 illustrates an example system 100 according to embodiments of the present invention. Embodiments of the invention allow parties 102 to determine whether they both hold information on the same person 104.

A party 102 may be any entity that holds one or more items of information (or data) on each of one or more data subjects 104. A party 102 may be a single person or an organisation such as a company (business), or an academic or state institution, etc. Examples of parties include, amongst others, a financial institution (e.g. commercial banks, investment banks, insurance providers, credit unions, stock brokers, asset management firms, insurance companies, finance companies, building societies), a health service provider (e.g. a general practice, a hospital, a dentist practice, an optician practice), an education provider (e.g. a school, a university), a judicial institution (e.g. a court), a government institution (e.g. a local council, foreign office, commonwealth office, passport provider, etc.), a utility service provider (e.g. an energy supplier, a water supplier), a television service provider, a telecommunications provider, and an internet service provider. Whilst Figure 1 shows two parties (first party 102a and second party 102b), it will be appreciated that there may be (many) more than two parties connected to a network (e.g. the Internet) 106.

A data subject 104 may be any identifiable subject that has one or more ascribed identifiers and has data held or accessible by one or more of the parties. For example, a data subject 104 may be a person (e.g. a customer, subscriber, user, student, consumer, patient, etc. of a relevant party 102). A data subject 104 may not necessarily be a person. For example, a data subject may be an object (e.g. a vehicle, a contract, a record such as, for example, a medical or education record, etc.). A data subject 104 may also be a company. Hereinafter, any example described for a person applies equally to any data subject 104, unless the context requires otherwise. Each data subject 104 is shown associated with a respective user device 108. A user device 108 may be a mobile user terminal such as a smartphone or tablet, or even a wearable device that can be worn about the user's person. The user device 108 may also be a television (e.g. a smart TV), a laptop, a computer or a games console. The user device 108 can be mains powered, battery powered, or use energy-harvesting techniques to supply its energy. Each user may have the same user device 108 or a different user device 108. Whilst Figure 1 shows three data subjects 104a, 104b, 104c it will be appreciated that there may be (many) more than three data subjects connected to the network 106.

The invention enables the determination of whether a data subject 104 having ascribed identifiers is known to two or more parties. An identifier may be ascribed to a data subject 104 by the data subject 104 themselves, by a party 102, by an official body, etc. The ascribed identifiers may be personal identifiers (e.g. a name). The ascribed identifiers may be unique to a data subject 104 (e.g. a passport number). Alternatively, the ascribed identifiers may not be unique to a data subject 104 (e.g. a shared address). Examples of identifiers include, amongst others, a first name or names, a surname, a date of birth, a nationality, a city of birth, an address, a passport number, a national insurance number, a driving license number, and a biometric identifier (e.g. photograph, voice recording, retina scan, fingerprint), vehicle make and/or model, vehicle license number, vehicle registration number, company registration number, contract number, medical record number, etc.

Each party 102 maintains, on their respective computer equipment, their own data record (or database) 114 comprising a plurality of entries. Hereinafter, the terms data record and database are synonymous unless the context requires otherwise. A data record includes databases, spreadsheets, file systems, individual files, a cloud database, a distributed database. Each entry in the data record 114 represents a data subject 104. Each entry representing a data subject 104 comprises one or more identifiers of that data subject 104 (i.e. the ascribed identifiers). That is, each party 102 stores at least one respective identifier (e.g. a name) for a set of data subjects (e.g. their customers, members, employees, students). Each party 102 may store its respective data record 114 in memory of its computing equipment and/or may access the data record 114 e.g. from a server 110 or from the cloud.

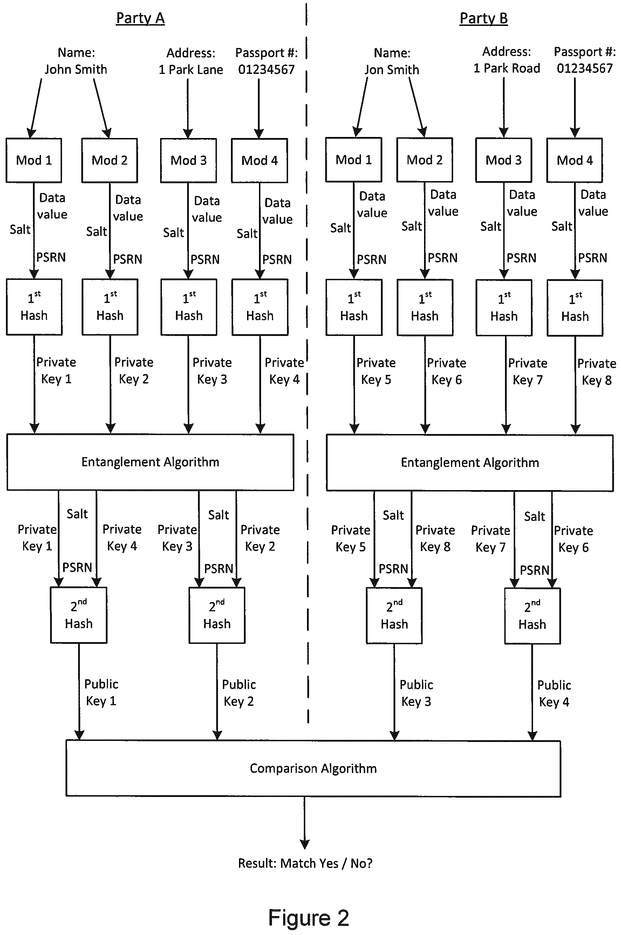

Figure 2 illustrates an example method for determining whether two parties (Party A and Party B) both have an entry in their respective databases 114 corresponding to the same person 104. As shown in Figure 2, Party A comprises an entry in their database for a person having a name identifier of "John Smith", an address identifier of "1 Park Lane", and a passport number identifier of "01234567". Party A comprises an entry in their database for a person having a name identifier of "Jon Smith", an address identifier of "1 Park Road", and a passport number of "01234567".

Each party 102 may compare entries in their databases 114 to determine whether two or more parties have a person 104 in common. To do this securely without having to divulge personal information, a first party 102a applies one or more modification algorithms to one or more identifiers of a person 104 corresponding to an entry in its database 114. One, some or all of the parties may apply the same said modification algorithm(s) to the same respective identifier(s). Parties may apply the modification algorithm(s) to one, some or all of the identifiers they hold for a given individual. The modification algorithms are provided to each party 102 applying said algorithms. For example, the match-maker 112 may provide the modification algorithms. The modification algorithms may be updated regularly (e.g. daily, weekly, monthly, etc.) or irregularly (e.g. upon request or at random intervals).

A modification algorithm (or a transformation algorithm) is any algorithm that, when applied to an identifier, modifies (or transforms) the identifier to generate at least one respective data value. For example, a modification algorithm may generate a single data value. Alternatively, a modification may generate multiple data values. In some examples, an algorithm may be applied to an identifier that does not modify the identifier. For instance, this algorithm, when applied to an identifier (e.g. a postcode), may return that identifier unchanged (e.g. the data value would be said postcode).

As shown in Figure 2, two modification algorithms (Mod 1 and Mod 2) are applied to the name identifiers held by Party A and Party B. Each party applies the same modification algorithm to the same type of identifier (and therefore generates the same data value). Likewise, the same modification algorithms (Mod 3 and Mod 4) are applied to the address identifier and passport number identifier respectively. The modification algorithms Mod 1-4 may be the same modification algorithms or different modification algorithms. Note that whilst the modification algorithms may be different, each party applies the same algorithm to the same identifier and consequently generates the same data value. E.g. party A and party B will apply a first same algorithm to a first same respective data value, but they may apply a second same algorithm to a second same respective data value. The first and second algorithms may be different. Each modification algorithm generates a respective data value.

Each modification algorithm may be configured to be applied to a particular type of identifier. For example, a first modification algorithm may be applied to a "name" identifier, whilst a second modification algorithm is instead applied to a "date of birth" identifier. More than one identifier may be applied to the same type of identifier. E.g. two or more modification algorithms may be applied to a "passport number" identifier.

In some embodiments, at least one of the modification algorithms may be a linguistic modification algorithm. A linguistic modification algorithm may modify an identifier containing some or only text (e.g. a name, and address, a nationality, a birthplace, etc.). For instance, a linguistic modification algorithm may remove certain letters from an identifier, e.g. the first and last letters, every third letter, every vowel, etc. Alternatively, a linguistic modification algorithm may re-arrange certain words or letters in an identifier, e.g. swapping the first and second letters, swapping the third and fourth letters, and so on.

In some embodiments, at least one of the modification algorithms may be a numerical modification algorithm. A numerical modification algorithm may modify an identifier containing some or only numbers (e.g. a phone number, a date of birth, a passport number, a national insurance number, etc.). For instance, a numerical modification algorithm may remove certain numbers (or digits from a number), add certain numbers (or digits from a number), swap certain numbers, etc. As another example, a numerical modification algorithm may modify one or more numbers in an identifier, e.g. doubling some or all numbers, raising some or all numbers to a power, etc.

In some embodiments, at least one of the modification algorithms may covert an identifier, such as, for example, an image, video, sound, etc. into a predefined formatted data value.

Each data value generated by one of the modification algorithms is individually input into a common hash function to generate a respective key (often referred to as a hash, a hash value, a hash code). The respective keys are shown as "Private Keys" in Figure 2. A hash function is any function that can be used to map data of arbitrary size (the data value) to data of a fixed size (the key). A hash function is a one-way function, that is, a function which is infeasible to invert. It is also deterministic, i.e. the same message always results in the same key. Furthermore, the hash function is configured such that a small change to the input results in an extensive change in the key such that the new key would appear uncorrelated with the old key. The same hash function is provided to each party 102, e.g. by the match-maker 112.

As shown in Figure 2, each data value is input into the same hash function ("1st Hash"). However, it is also possible for each type of identifier to be assigned its own hash function, which may differ to the hash function of at least one other, different type of identifier. E.g. the data values generated by applying Mod 1 and Mod 2 to the name identifier may be input to a hash function that is different to the data value generated by applying Mod 3 to the address identifier. However, even if different hash functions are used for different identifiers, each party employs the same hash function for those different identifiers.

Any hash function may be used in the described embodiments. For example, the hash function may be a SHA-224, SHA-256, SHA-384, or a SHA-512 hash function. Other (cryptographic) hash functions will be known to the skilled person.

Each party 102 may store the keys that it has generated, by inputting the data values into a hash function, in a key list. For example, the key list may be stored in memory at a respective party's server 110.

It is often the case that different parties 102 may hold (slightly) different versions of the same identifier for their respective plurality of persons 104. For example, identifiers may be stored in different formats, e.g. a full name may or may not include a middle name, a date of birth may or may not include hyphens, forward slashes, etc. As another example, identifiers may be stored with discrepancies, or errors, e.g. spelling errors. Errors or formatting differences, amongst others, can be introduced due to a person 104 (unintentionally) providing erroneous information, a party 102 taking down erroneous information, e.g. when collecting information over the phone, etc. Therefore an advantage of applying modification algorithms to the identifiers held by each party 102 is that errors or differences can be removed which enables identical keys to be produced. For instance, two parties may each hold a full name of an individual, e.g. "John Smith". However, the second party 102b collected the information over the phone and incorrectly entered said individual's name into their database 114 as "Jon Smith". If the first party 102a and the second party 102b did not apply a modification algorithm to the individual's name, they would not generate the same key when inputting the identifier to the common hash function. However, if a modification algorithm (e.g. a linguistic modification algorithm) is applied to the identifiers "John Smith" and "Jon Smith", which, say, removes the vowels from the identifier, both identifiers become "Jhn Smth". These identical data values would therefore generate identical keys.

A given party (e.g. the first party 102a) may supply their respective key set to a comparison algorithm. At least one other party (e.g. the second party 102b) may supply their respective key set to the comparison algorithm. The comparison algorithm receives, as inputs, at least a first and second key list from the first and second party 102a, 102b respectively, and determines whether the person 104 from which the respective key sets are generated corresponds to an entry in both the first party's database 114a and the second party's database 114b, i.e. whether the person 104 is common to both the first and second party 102a, 102b. The comparison algorithm compares one or more of the respective keys in the first key set with one or more respective keys in the second key set generated by the first party 102b. The comparison algorithm may compare all of the keys in the first key set with all of the keys in the second key set to search for any matches. The comparison algorithm then determines whether the compared key sets comprise at least one identical key. In some examples, the comparison algorithm may determine whether the key sets comprise more than one identical keys, e.g. whether all of the keys in a first set are present in the second key set and/or vice versa. Each participant may receive a result of the comparison algorithm which indicates whether the person 104 is common to the first and second party 102a, 102b. The result may not be binary. That is, the result may not necessarily indicate a definite match in data subjects 104. Instead, the result may indicate a likelihood (or probability) of a match in data subjects 104. For example, the result may state that there is a 70% that the data subject 104 is common to the first and second party 102a, 102b.

In the example of Figure 2, additional steps are performed before supplying keys to the comparison algorithm. However, in some embodiments of the invention these steps are omitted.

Due to the properties of a hash function, i.e. only the same single input can produce a given output, if the key sets comprise one or more identical keys between them, this means that the parties have an identical identifier for a given individual. Depending on the identifier, a single matching key may be enough to indicate that the person 104 is common to both parties. For example, a matching key derived from a passport number may indicate a matching individual. In contrast, a matching key derived from an address may not necessarily indicate a matching person 104 as an address may be shared.

The result of the comparison, i.e. whether the person 104 corresponds to an entry in both the database 114 of the first party 102a and the second party 102b may be based on the number of identical keys between the compared key sets being greater than a threshold. The threshold may be zero. That is, a single identical key may indicate a matching individual. Alternatively, the threshold may be greater than zero, e.g. one, ten, twenty, etc. The threshold may be less than the total number of keys in a given key set. That is, a match may be determined even if not all of the keys in a first party's key set are also included in a second party's key set. The threshold may be predetermined by one or more of the parties (e.g. the first party 102a) or by the match-maker 112. For example, a given party 102 may decide that they will only acknowledge a common person 104 if the compared key sets comprise at least ten identical keys. The threshold may be updated by a party 102 of by the match-maker 112. In some examples, the threshold may be a percentage. For example, the threshold may be 70%, e.g. 70% of keys in the first key set must match with keys in the second key set.

In some embodiments, the comparison algorithm is internal to the computing equipment of at least one of the parties. For example, the comparison algorithm may be performed by the first party's computing equipment. In this example, the second party 102b (or parties) transmits its respective key list to the first party 102a for the first party 102a to input both key lists to the comparison algorithm. Additionally or alternatively, the comparison algorithm may be performed by the second party's computing equipment, with the first party 102a transmitting its key list to the first party 102b. More generally, each party 102 may transmit their respective key list to one or more different parties. Similarly, each party 102 may receive a respective key list from one or more different parties. Each party 102 may comprise an internal comparison algorithm. The key list(s) may be transmitted to a party 102 directly (e.g. over the network) or via the match-maker 112.

In other embodiments, the comparison algorithm is external to the parties. For example, the comparison algorithm may be performed by the match-maker's computing equipment. In this example, each party 102 may supply (i.e. transmit) its respective key list to the match-maker 112 who performs the comparison algorithm. In some examples, the match-maker 112 may not perform the comparison algorithm. Instead, the match-maker 112 may simply publish the first and/or second sets to one, some or all of the parties. In other examples, the match-maker 112 may perform the comparison and also publish the key set(s). The match-maker 112 may transmit the result of the comparison to one or more of the parties.

Publishing the key sets allows each party 102 to determine whether they have a person 104 common to another party 102. That is, if a first party 102a has derived a set of keys and a first party 102b has derived an identical set of keys (or at least one or more identical keys), both parties can be assured that they have a common individual, as the keys must have been generated from the same identifiers of the individual.

In some embodiments, the keys generated by the common hash function are not transmitted from one party 102 to another party 102, or from one party 102 to the match-maker 112. In these embodiments, the keys generated by the (first) common hash function are private keys (or hidden keys) and are not shared. Instead, two or more private keys are combined and input to a second common hash function to generate a respective public key. E.g. in the example of Figure 2, private key 1 and private key 4 are input to a 2$^{nd}$ hash function, whilst private key 3 and private key 2 are separately input into a 2$^{nd}$ hash function. The two or more private keys may be combined by concatenation or otherwise, but the particular method of combination is pre-defined and common to both parties. The particular private keys selected to be combined and input into the second hash function are chosen by an entanglement algorithm, as shown in Figure 2 and discussed below. The first and second common hash function are provided to each party 102, e.g. by the match-maker 112. The first and second common hash functions may or may not be the same hash function as the first common hash function.

A private key generated from a first identifier (e.g. an address) may be combined with a second, different identifier (e.g. a driving license number) from a different category of identifiers. An advantage of this is that if two compared key sets comprising public keys are compared and an identical public key is identified, the parties can be more certain that they have a person 104 in common. This is due to the nature of the hash function, i.e. the chances of a hash of multiple hashes matching the hash of multiple different hashes is miniscule and therefore the data values that produced the two matching public keys must be identical (and therefore very likely to have been produced by at least near-identical identifiers). A further advantage is that the likelihood of reverse engineering the public key is even smaller than the likelihood of reverse engineering the private key, therefore increasing the security of the individual's identifiers.

Instead of the private key sets (i.e. the sets of generated private keys) being compared, the public key sets (i.e. the sets of generated public keys) are supplied to the comparison algorithm for comparison. Again, the comparison algorithm compares one or more keys in a first public key set generated by a first party 102a with one or more keys in a second public key set generated by a first party 102b and determines whether the compared key sets comprise one or more identical public keys. The result of the determination is provided to one or more parties. The public keys may be published by one or more parties and/or by the match-maker 112.

For example, a public key may be published along with information associated with the person 104 from which the public key is generated. For example, the public key may "X3GT" and the information may indicate that the person 104 (e.g. John) has paid their recent energy bill. In this way, information about a person 104 can be shared without ever having to share information that personally identifies John. If a different party 102 has generated the same public key, they can tell that John has paid his most recent energy bill.

When inputting the private keys to the second common hash function, the private keys may be selected based on a common entanglement algorithm, as shown in Figure 2. The common entanglement algorithm may be provided to each party 102, e.g. by the match-maker 112. The entanglement algorithm may choose one or more private keys, or parts of private keys, generated from one or more respective identifiers to input to the second common hash function. I.e. a public key may be generated from the hash of two or more private keys, or parts of private keys, with each private key being generated from a different type of identifier (e.g. name, nationality, etc.). The entanglement algorithm dictates the number of private keys, or parts of private keys, input to the second common hash function and the particular private keys, or parts of private keys, that are input to said second common hash function. In the example of Figure 2, the entanglement algorithm selects two private keys to be input to each of the second hash functions ("2$^{nd}$ Hash"). Each party inputs the private keys that have been generated by applying the same modification algorithm to the same type of identifier to the same hash function. For example, Party A applies Mod 1 to the name identifier and inputs the resulting data value to 1$^{st}$ Hash to generate private key 1. Similarly, Party B applies Mod 1 to the name identifier and inputs the resulting data value to 1$^{st}$ Hash to generate private key 5. Party A applies Mod 4 to the passport number identifier and inputs the resulting data value to 1$^{st}$ Hash to generate private key 4. Similarly, Party B applies Mod 4 to the passport number identifier and inputs the resulting data value to 1$^{st}$ Hash to generate private key 8. Party A then inputs private keys 1 and 4 to 2$^{nd}$ Hash to generate public key 1. Similarly, Party B then inputs private keys 5 and 8 to 2$^{nd}$ Hash to generate public key 3. The entanglement algorithm further increases the likelihood of a matching public key corresponding to a matching individual.

As an optional feature, each respective data value may be combined with a cryptographic salt, and the data value is input to the first common hash function along with the combined cryptographic salt to generate the private key. Each respective data value is combined with the same salt, that same salt being provided to each of the parties. That is, if the data value is a first name, each party is provided the same salt to combine with the first name of the data subject 104. Figure 2 shows each data value being input to a hash function with a combined salt. A cryptographic salt (or salt) is a (random) value that is used as an additional input to a hash function that to defend against dictionary attacks, rainbow table attacks, etc. Due to the salt being input to the hash function, a different salt will lead to the hash function generating a substantially different key. Therefore, not only does the salt make the process more secure, it also ensures that two parties can only produce an identical key if they combine the same salt to a data value when generating a key.

Each type (or kind, category, etc.) of data value may be assigned its own salt, with each salt being different for each data value. Alternatively, each set of data values generated by applying a specific set of modification algorithms to a given identifier may be assigned the same salt, or each set of data values generated by applying a set of modification algorithms to the same identifier may be assigned a different salt. As another example, each person 104 may be assigned his/her own salt. A salt may be combined with a data value by concatenation or otherwise. To ensure that the process is replicable by all parties, each party 102 must assign the same salt to the same type of data values (i.e. the same data values used to generate a private key, which may or may not be identical data values depending on whether the two parties have a common person 104 or the same identifiers for an individual).

Additionally or alternatively, a cryptographic salt may be combined with the private hashes input to the second common hash function to generate the public key. Figure 2 shows each private key being input to a hash function with a combined salt

In some embodiments, each party 102 is provided with a common seed number, e.g. from the match-maker 112. Each party 102 receives the same common seed number for a respective data value in order to search for a pseudorandom number (often called a nonce) that solves a respective hash function. The seed number may be input into the first and/or second common hash functions to generate the private and/or public keys respectively. Each party 102 may receive a single seed number to be input to both hash functions, or they may receive separate seed numbers to determine separate pseudorandom numbers be input to the first and second common hash functions. The use of the same seed and the same input (e.g. data value or private key, and optionally a salt) results in finding the same pseudorandom number that solves the hash. The seed number is an arbitrary number that, when input into the hash function (e.g. combined with the data value or private key and, optionally, the salt), generates a key that meets a predefined criteria. For example, the criteria may be a threshold that the generated key must be above or below. As another example, the criteria may be a predetermined starting sequence of numbers, e.g. the key must start with four zeros. Due to a cryptographic hash function being a "one-way" function, there is no way to reverse engineer the function to calculate a nonce value. This further improves the security of the process, preventing an individual's identifier from being discovered.

Depending on the required criteria, more than one pseudorandom number may generate a key that meets the criteria. That is, there may be a sequence of pseudorandom numbers that, when input to a hash function along with a data value, generates a key that meets the criteria (e.g. a number less than a certain value). In some examples, each party 102 may be required to find from within this sequence the first pseudorandom number greater than the seed number that result in the criteria being met.

Alternatively, each party 102 may be required to find the first pseudorandom number less than the seed number that result in the criteria being met. This way, only two parties that have the same seed number and instructions (e.g. find the first number greater than the seed number that results in the key starting with two zeros) will generate a key by finding the same pseudorandom number. The seed number and/or criteria may be depending on the person 104 and/or the identifier. E.g. a personal identifier (e.g. home address) may be assigned a seed number and criteria combination such that the criteria is harder to be fulfilled, and vice versa for a non-personal identifier (e.g. last three digits of a phone number). Figure 2 illustrates each data value being input to a hash function with a pseudorandom number ("PSRN" in Figure 2). Similarly, Figure 2 illustrates each private key being input to a hash function with a PSRN.

The seed number may be updated, e.g. for security purposes. The seed number may be updated periodically (e.g. minutely, hourly, daily, weekly), randomly, upon request (e.g. from one of the parties) and/or in response to a criteria being met. The seed number may be updated by the match-maker 112, i.e. the match-maker 112 transmits a new seed number to each party 102. An example of a criteria being met may be, for example, a new person 104 being checked for commonality between the parties.

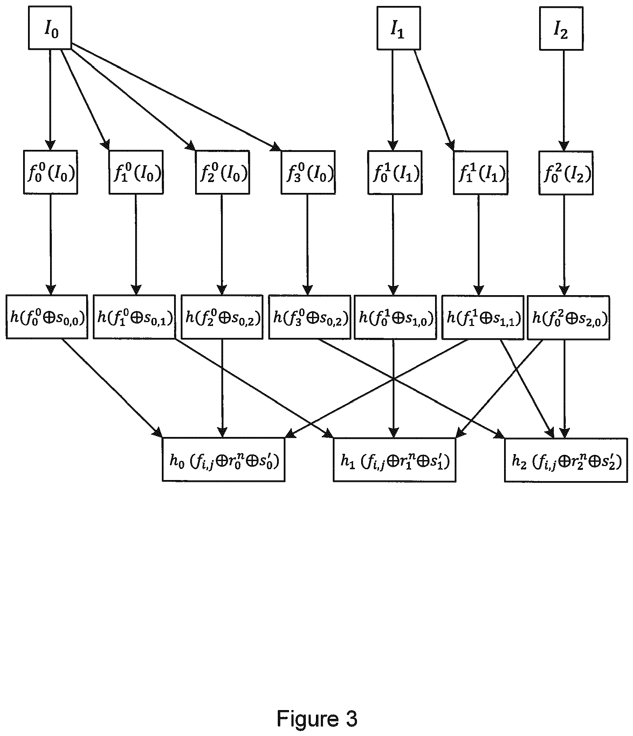

Figure 3 illustrates an example method for generating private and public keys. In this example, a party 102 maintains a database 114 in which an entry corresponding to a person 104 comprises at least three different identifiers for that person 104. The three identifiers are $I_0$ (e.g. first name), $I_i$ (e.g. surname) and $I_z$ (e.g. email address). Modification algorithms (e.g. $f_0^0$) are then applied to the identifiers. A plurality of modification algorithms may be applied to a given identifier. For example, four modification algorithms $f$ are applied to identifier $I_0$, two modification algorithms are applied to identifier $I_i$ and one modification algorithm f is applied to identifier $I_2$. The modification algorithms applied to different identifiers may be the same or different. In the example of Figure 2, four different modification algorithms are applied to identifier $I_0$, as indicated by the respective subscripts.

Then, a salt (e.g. $s_{0,0}$) is applied to each data value and the combined data value and salt are input to a common hash function. Each data value may be combined with the same salt or a different salt. Each data value generated from a respective identifier may be combined with the same salt, with each data value generated from a different respective identifier being combined with a different, same (i.e. common) salt. The output of the common hash function is a private key. As shown in Figure 2, the seven data values produced from the three identifiers are used to generate seven respective private keys.

During entanglement, two or more private keys are input into a common hash function to generate a respective public key. The private keys that are input to the common hash function are predetermined. For example, as shown in Figure 2, three private keys are input to a hash function $h_0$ to generate a respective private key, one private key stemming from each of the three identifiers. Similarly, three different private keys are input to hash function $h_1$ and three different private keys are input to hash function $h_2$. Along with the two or more private keys, a salt (e.g. $s_0'$) may also be input to the hash function to generate a private key, as discussed above. Furthermore, each hash function may take, as an input, a pseudorandom number (e.g. $r_0^n$).

In summary, embodiments of the present invention use a combination of algorithmic techniques to build a cryptographic key specific to each individual. The key is built by applying these algorithms to the individual's own identifiers. Consequently, if two or more parties generate the same key they can be sure that they have entries in their respective databases 114 corresponding to the same person 104, and can thus share information regarding that person 104 without ever sharing that person's identity.

Returning to Figure 1, each person 104 may provide one or more of the parties with data which is stored in memory (e.g. in a database 114) by the respective party 102. For example, a person 104 may provide data to a party 102 via their user device 108. Similarly, each person 104 may provide one or more identifiers to one or more respective parties, those identifiers being stored in a database 114 by the respective party 102. The user device 108 may comprise a user interface arranged to receive an input from the user (e.g. data and/or identifier(s)) and operatively coupled to a controller. The user interface may comprise a display in the form of a screen and some arrangement for receiving inputs from the user. For example, the user interface may comprise a touch screen, or a point-and-click user interface comprising a mouse, track pad, microphone for detecting voice input, in-air gesture sensor or tracker ball or the like. Alternatively, information and/or identifiers may be provided to a party 102 from a person 104 without use of their user device 108, e.g. in person or via post.

The controller of the user device 108 may also be coupled to the network via a wireless transceiver. For example, the wireless transceiver may communicate with the network via any suitable wireless medium, e.g. a radio transceiver for communicating via a radio channel. Alternatively, the wireless transceiver may communicate with the network via a local area network such as a WLAN or a wide area network, such as the internet. Alternatively, the user devices may each comprise a wired connection to the network, e.g. an Ethernet or DMX connection.

Each party 102 employs respective computer equipment to store its respective database 114 and perform its respective method. For example, the computing equipment employed by each party 102 may comprise some or all of the resources of a respective server 110a, 110b connected to the network, the server 110 comprising one or more server units at one or more geographic sites for storing data provided by or collected from the individual. It is also not excluded that the servers 110 may be virtual servers (servers implemented by means of different secure enclaves operated on some or all of the same physical hardware). Note that a person's data may also be provided by a third party match-making service. In embodiments the functionality of the computing equipment and server 110 is implemented in the form of software stored in memory and arranged for execution on a processor (the memory on which the software is stored comprising one or more memory units employing one or more storage media, e.g. EEPROM or a magnetic drive, and the processor on which the software is run comprising one or more processing units). Alternatively it is not excluded that some or all of the functionality of the computing equipment and server 110 could be implemented in dedicated hardware circuitry, or configurable or reconfigurable hardware circuitry such as an ASIC or a PGA or FPGA. Each party (e.g. their server 110) may be connected to the network via a local area network such as a WLAN or a wide area network, such as the internet. Alternatively, the respective computer equipment of each party 102 may each comprise a wired connection to the network, e.g. an Ethernet or DMX connection.

Figure 1 also shows a third-party "match-maker" (or "match-making service") 112. The match-maker 112 employs computer equipment to perform its respective method. For example, the computing equipment employed by the match-maker 112 may comprise some or all of the resources of a server 110c connected to the network, the server comprising one or more server units at one or more geographic sites for storing data provided by or collected from the parties. It is also not excluded that the servers may be virtual servers (servers implemented by means of different secure enclaves operated on some or all of the same physical hardware). In embodiments the functionality of the computing equipment and server 110c is implemented in the form of software stored in memory and arranged for execution on a processor (the memory on which the software is stored comprising one or more memory units employing one or more storage media, e.g. EEPROM or a magnetic drive, and the processor on which the software is run comprising one or more processing units). Alternatively it is not excluded that some or all of the functionality of the computing equipment and server 110 could be implemented in dedicated hardware circuitry, or configurable or reconfigurable hardware circuitry such as an ASIC or a PGA or FPGA. The match-maker (e.g. server) 112 may be connected to the network via a local area network such as a WLAN or a wide area network, such as the internet. Alternatively, the computing equipment of the match-maker 112 may each comprise a wired connection to the network, e.g. an Ethernet or DMX connection.

It will be appreciated that the above embodiments have been described by way of example only. Other applications or variants of the disclosed techniques may become apparent to a person skilled in the art once given the disclosure herein. The scope of the present disclosure is not limited by the above-described embodiments but only by the accompanying claims.

**Claims**

1. A method of comparing data record entries of two or more parties (102), wherein each party (102) maintains a data record comprising a plurality of entries, each entry representing a corresponding data subject (104) and comprising one or more identifiers of that data subject (104), wherein the method comprises operating computing equipment of a first one of the two or more parties to perform operations of:

   to each of one or more of the identifiers of the data subject corresponding to one of the entries in the data record of the first party (102a), applying one or more common modification algorithms common to the two or more parties (102), wherein each modification algorithm modifies the identifier to which it is applied to thereby generate a respective data value;
   for each generated data value, inputting at least that data value to a common hash function common to the two or more parties in order to generate a respective key, wherein said generating of the respective key comprises:

      for each generated data value, inputting at least that data value to a first common hash function to generate a respective private key;
      inputting one or more respective private keys generated by the first party (102a) to a second common hash function common to the two or more parties in order to generate a respective public key, wherein the one or more respective private keys input to the second common hash function are chosen based on a common entanglement algorithm common to the two or more parties, wherein the common entanglement algorithm prescribes which of the generated private keys and/or which parts of the generated private keys are input to the second common hash function;

   storing each generated key in a first key set, wherein said storing comprises storing at least each generated public key in the first key set;
   supplying the first key set to a comparison algorithm configured to determine whether said one of the data subjects corresponds to an entry in both the data record of the first party (102a) and a data record of a second party (102b), wherein the first and second parties are different ones of the two or more parties by: (i) comparing one or more of the keys in the first key set with one or more keys in a second key set generated by the second party, and (ii) determining whether the compared key sets comprise one or more identical respective keys, wherein said comparing comprises comparing one or more of the public keys in the first key set with one or more public keys in the second key set generated by the second party (102b), and wherein said determining comprises determining whether the compared key sets comprises one or more identical respective public keys; and
   receiving a result of the comparison algorithm, wherein the result indicates whether said one of the data subjects corresponds to an entry in both the data record of the first party (102a) and the data record of the second party (102b).

2. A method according to claim 1, wherein the result indicates a likelihood of whether said one of the data subjects corresponds to an entry in both the data record of the first party (102a) and the data record of the second party (102b).

3. A method according to claim 1 or claim 2, wherein the comparison algorithm is configured to determine whether said one of the data subjects corresponds to an entry in both the data record of the first party (102a) and the data record of the second party (102b) by determining whether the compared key sets comprise a number of identical keys greater than or equal to a threshold number.

4. A method according to any preceding claim, wherein the comparison algorithm is an internal comparison algorithm performed by the computing equipment of the first party (102a), and wherein said supplying comprises receiving the second key set from the second party (102b) and supplying the receiving second key set to the internal comparison algorithm.

5. A method according to any of claims 1 to 4, wherein the comparison algorithm is an external comparison algorithm performed by computing equipment of a third party match-making service (112) external to the two or more parties, and wherein said supplying comprises transmitting the first key set to said match-making service.

**6.** A method according to any preceding claim, comprising:

receiving a common seed number common to the two or more parties for determining a common pseudorandom number; and
wherein said inputting comprises inputting the common seed number to at least one of the hash functions to generate the respective key.

**7.** A method according to claim 6, comprising updating the seed number used to determine the common pseudorandom number.

**8.** A method according to any preceding claim, comprising:

for each of said data values, combining that data value with a same respective cryptographic salt common to the two or more parties for that data value; and
wherein said inputting of at least the respective data value to the first hash function comprises inputting at least the respective data value with the combined cryptographic salt to the first hash function to generate the private key.

**9.** A method according to claim 6 and claim 8, wherein said inputting comprises inputting at least the respective data value with the combined cryptographic salt and the determined pseudorandom number to at least one of the hash functions to generate the respective key.

**10.** A method according to any preceding claim, wherein the one or more modification algorithms comprise one or more linguistic modification algorithms and/or one or more numerical modification algorithms.

**11.** A method according to any preceding claim, wherein the one or more identifiers comprise at least one of: a first name, a surname, a date of birth, a nationality, a city of birth, an address, a passport number, a national insurance number, a driving license number, a vehicle registration number, a company registration number, a contract number, an internet protocol address, and/or a biometric identifier.

**12.** A method according to any preceding claim, wherein said applying comprises applying a plurality of modification algorithms to the at least one of the identifiers of the data subject corresponding to one of the entries in the data record of the first party (102a).

**13.** A method of comparing data record entries of two or more parties (102), wherein each party (102) maintains a data record comprising a plurality of entries, each entry representing a corresponding data subject (104) and comprising one or more identifiers of that data subject (104), wherein the method comprises operating computing equipment of a third-party match-making service (112) other than the two or more parties to perform operations of:

providing one or more common modification algorithms to each of the two or more parties (102), wherein each modification algorithm modifies the identifier to which it is applied to thereby generate a respective data value;
providing one or more common hash functions to each of the two or more parties (102), wherein each common hash function, when applied to the respective data value, generates a respective key, wherein said providing of the common hash functions comprises:

providing a first common hash function to each of the two or more parties (102), wherein the first common hash function, when applied to the respective data value, generates a respective private key; and
providing a second common hash function to each of the two or more parties (102), wherein the second common hash function, when applied to one or more of the private keys generated each party, generates a respective public key, and

wherein the method further comprises:

providing a common entanglement algorithm to each of the two or more parties (102), wherein the entanglement algorithm prescribes which of the generated private keys and/or which parts of the generated private keys are input to the second common hash function;
receiving a first key set from a first one of the two or more parties, wherein the first key set comprises keys generated by the first party (102a);
receiving a second key set from a second one of the two or more parties, the first party (102a) and second party (102b) being different ones of the two or more parties, wherein the second key set comprises keys generated by the second party (102b);
publishing the first and/or second key set to one or more of the two or more parties (102);

comparing one or more of the keys of the first key set with one or more of the keys of the second key set;

determining whether said one of the data subjects corresponds to an entry in both the data record of the first party (102a) and the data record of the second party (102b) based on whether the compared key sets comprise one or more identical respective keys; and

transmitting a result of said determination to the first and/or second party, wherein the result indicates whether said one of the data subjects corresponds to an entry in both the data record of the first party (102a) and the data record of the second party (102b).

14. A computer program for comparing data record entries of two or more parties (102), wherein each party (102)maintains a data record comprising a plurality of entries, each entry representing a corresponding data subject (104) and comprising one or more identifiers of that data subject (104); wherein the computer program comprises instructions embodied on computer-readable storage and configured so as, when the program is executed by a computer, cause the computer to perform operations of:

to each of one or more of the identifiers of the data subject (104) corresponding to one of the entries in the data record of the first party (102a), applying one or more common modification algorithms, wherein the one or more modification algorithms are common to the two or more parties, wherein each modification algorithm modifies the identifier to which it is applied to thereby generate a respective data value;

for each generated data value, inputting at least that data value to a common hash function common to the two or more parties in order to generate a respective key, wherein said generating of the respective key comprises:

for each generated data value, inputting at least that data value to a first common hash function to generate a respective private key;

inputting one or more respective private keys generated by the first party (102a) to a second common hash function common to the two or more parties in order to generate a respective public key, wherein the one or more respective private keys input to the second common hash function are chosen based on a common entanglement algorithm common to the two or more parties, wherein the common entanglement algorithm prescribes which of the generated private keys and/or which parts of the generated private keys are input to the second common hash function;

storing each generated keys in a first key set, wherein said storing comprises storing at least each generated public key in the first key set;

supplying the first key set to a comparison algorithm configured to determine whether said one of the data subjects corresponds to an entry in both the data record of the first party (102a) and a data record of a second party (102b), wherein the first and second parties are different ones of the two or more parties by: (i) comparing one or more of the keys in the first key set with one or more keys in a second key set generated by the second party, and (ii) determining whether the compared key sets comprise one or more identical respective keys, wherein said comparing comprises comparing one or more of the public keys in the first key set with one or more public keys in the second key set generated by the second party, and wherein said determining comprises determining whether the compared key sets comprises one or more identical respective public keys; and

receiving a result of the comparison algorithm, wherein the result indicates whether said one of the data subjects corresponds to an entry in both the data record of the first party and the data record of the second party.

15. A computer program for comparing data record entries of two or more parties (102), wherein each party (102) maintains a data record comprising a plurality of entries, each entry representing a corresponding data subject (104) and comprising one or more identifiers of that data subject; wherein the computer program comprises instructions embodied on computer-readable storage and configured so as, when the program is executed by a computer of a third-party match-making service (112) other than the two or more parties, cause the computer to perform operations of:

providing one or more common modification algorithms to each of the two or more parties (102), wherein each modification algorithm modifies the identifier to which it is applied to thereby generate a respective data value; and

providing one or more common hash functions to each of the two or more parties (102), wherein each common hash function, when applied to the respective data value, generates a respective key, wherein said providing of the common hash functions comprises:

providing a first common hash function to each of the two or more parties (102), wherein the first common hash function, when applied to the respective data value, generates a respective private key; and providing a second common hash function to each of the two or more parties (102), wherein the second common hash function, when applied to one or more of the private keys generated each party, generates a respective public key, and wherein the operations further comprise:

providing a common entanglement algorithm to each of the two or more parties (102), wherein the entanglement algorithm prescribes which of the generated private keys and/or which parts of the generated private keys are input to the second common hash function; receiving a first key set from a first one of the two or more parties, wherein the first key set comprises keys generated by the first party (102a); receiving a second key set from a second one of the two or more parties, the first party (102a) and second party (102b) being different ones of the two or more parties, wherein the second key set comprises keys generated by the second party (102b); publishing the first and/or second key set to one or more of the two or more parties (102); comparing one or more of the keys of the first key set with one or more of the keys of the second key set; determining whether said one of the data subjects (104) corresponds to an entry in both the data record of the first party (102a) and the data record of the second party (102b) based on whether the compared key sets comprise one or more identical respective keys; and transmitting a result of said determination to the first and/or second party, wherein the result indicates whether said one of the data subjects corresponds to an entry in both the data record of the first party (102a) and the data record of the second party (102b).

**Patentansprüche**

1.  Verfahren zum Vergleichen von Datensatzeinträgen von zwei oder mehr Parteien (102), wobei jede Partei (102) einen Datensatz mit mehreren Einträgen führt, wobei jeder Eintrag ein entsprechendes Datensubjekt (104) darstellt und ein oder mehr Kennungen dieses Datensubjekts (104) aufweist, wobei das Verfahren ein Betreiben einer Computerausrüstung einer ersten der zwei oder mehr Parteien aufweist, um Operationen durchzuführen von:

auf jeden von ein oder mehr der Kennungen des Datensubjekts, die einem der Einträge im Datensatz der ersten Partei (102a) entsprechen, Anwenden von ein oder mehr gemeinsamen Modifikationsalgorithmen, die den zwei oder mehr Parteien (102) gemeinsam sind, wobei jeder Modifikationsalgorithmus die Kennung modifiziert, auf die sie angewendet wird, um dadurch einen entsprechenden Datenwert zu erzeugen; für jeden erzeugten Datenwert Eingeben wenigstens dieses Datenwerts in eine gemeinsame Hash-Funktion, die den zwei oder mehr Parteien gemeinsam ist, um einen entsprechenden Schlüssel zu erzeugen, wobei das Erzeugen des entsprechenden Schlüssels aufweist:

für jeden erzeugten Datenwert Eingeben wenigstens dieses Datenwerts in eine erste gemeinsame Hash-Funktion, um einen entsprechenden privaten Schlüssel zu erzeugen; Eingeben von ein oder mehr der von der ersten Partei (102a) erzeugten entsprechenden privaten Schlüsseln in eine zweite gemeinsame Hash-Funktion, die den zwei oder mehr Parteien gemeinsam ist, um einen entsprechenden öffentlichen Schlüssel zu erzeugen, wobei die ein oder mehr in die zweite gemeinsame Hash-Funktion eingegebenen entsprechenden privaten Schlüssel basierend auf einem gemeinsamen Verschränkungsalgorithmus ausgewählt werden, der den zwei oder mehr Parteien gemeinsam ist, wobei der gemeinsame Verschränkungsalgorithmus vorschreibt, welche der erzeugten privaten Schlüssel und/oder welche Teile der erzeugten privaten Schlüssel in die zweite gemeinsame Hash-Funktion eingegeben werden;

Speichern jedes erzeugten Schlüssels in einem ersten Schlüsselsatz, wobei das Speichern ein Speichern wenigstens jedes erzeugten öffentlichen Schlüssels im ersten Schlüsselsatz aufweist; Liefern des ersten Schlüsselsatzes zu einem Vergleichsalgorithmus, der konfiguriert ist, um zu bestimmen, ob das eine der Datensubjekte einem Eintrag in sowohl dem Datensatz der ersten Partei (102a) als auch einem Datensatz einer zweiten Partei (102b) entspricht, wobei die ersten und zweiten Parteien unterschiedliche der zwei oder mehr Parteien sind, durch: (i) Vergleichen von ein oder mehr der Schlüssel im ersten Schlüsselsatz mit ein oder mehr Schlüsseln in einem zweiten von der zweiten Partei erzeugten Schlüsselsatz, und (ii) Bestimmen, ob die verglichenen Schlüsselsätze ein oder mehr identische entsprechende Schlüssel aufweisen, wobei das Vergleichen ein Vergleichen von ein oder mehr der öffentlichen Schlüssel im ersten Schlüsselsatz mit ein oder mehr öffentlichen Schlüsseln im von der zweiten Partei (102b) erzeugten zweiten Schlüsselsatz aufweist, und wobei das Bestimmen ein Bestimmen aufweist, ob die verglichenen Schlüsselsätze ein oder mehr identische entsprechende öffentliche Schlüssel aufweisen; und Empfangen eines Ergebnisses des Vergleichsalgorithmus, wobei das Ergebnis angibt, ob das eine der Datensubjekte einem Eintrag in sowohl dem Datensatz der ersten Partei (102a) als auch dem Datensatz der zweiten Partei (102b) entspricht.

2. Verfahren nach Anspruch 1, wobei das Ergebnis eine Wahrscheinlichkeit angibt, ob das eine der Datensubjekte einem Eintrag in sowohl dem Datensatz der ersten Partei (102a) als auch dem Datensatz der zweiten Partei (102b) entspricht.

3. Verfahren nach Anspruch 1 oder Anspruch 2, wobei der Vergleichsalgorithmus konfiguriert ist, um zu bestimmen, ob das eine der Datensubjekte einem Eintrag in sowohl dem Datensatz der ersten Partei (102a) als auch dem Datensatz der zweiten Partei (102b) entspricht, indem er bestimmt, ob die verglichenen Schlüsselsätze eine Anzahl identischer Schlüssel aufweisen, die größer oder gleich einer Schwellenzahl ist.

4. Verfahren nach einem der vorhergehenden Ansprüche, wobei der Vergleichsalgorithmus ein interner Vergleichsalgorithmus ist, der von der Computerausrüstung der ersten Partei (102a) durchgeführt wird, und wobei das Liefern ein Empfangen des zweiten Schlüsselsatzes von der zweiten Partei (102b) und ein Liefern des empfangenden zweiten Schlüsselsatzes zum internen Vergleichsalgorithmus aufweist.

5. Verfahren nach einem der Ansprüche 1 bis 4, wobei der Vergleichsalgorithmus ein externer Vergleichsalgorithmus ist, der von einer Computerausrüstung eines Drittpartei-Übereinstimmungsdienstes (112) außerhalb der zwei oder mehr Parteien durchgeführt wird, und wobei das Liefern ein Übertragen des ersten Schlüsselsatzes an den Übereinstimmungsdienst aufweist.

6. Verfahren nach einem der vorhergehenden Ansprüche, aufweisend:

Empfangen einer gemeinsamen Seed-Zahl, die den zwei oder mehr Parteien gemeinsam ist, zum Bestimmen einer gemeinsamen Pseudozufallszahl; und wobei das Eingeben ein Eingeben der gemeinsamen Seed-Zahl in wenigstens eine der Hash-Funktionen aufweist, um den jeweiligen Schlüssel zu erzeugen.

7. Verfahren nach Anspruch 6, aufweisend ein Aktualisieren der Seed-Zahl, die verwendet wird, um die gemeinsame Pseudozufallszahl zu bestimmen.

8. Verfahren nach einem der vorhergehenden Ansprüche, aufweisend:

für jeden der Datenwerte Kombinieren dieses Datenwerts mit einem gleichen jeweiligen kryptografischen Salz, das den zwei oder mehr Parteien für diesen Datenwert gemeinsam ist; und wobei das Eingeben von wenigstens dem entsprechenden Datenwert in die erste Hash-Funktion ein Eingeben von wenigstens dem entsprechenden Datenwert mit dem kombinierten kryptografischen Salz in die erste Hash-Funktion aufweist, um den privaten Schlüssel zu erzeugen.

9. Verfahren nach Anspruch 6 und Anspruch 8, wobei das Eingeben ein Eingeben von wenigstens dem jeweiligen Datenwert mit dem kombinierten kryptografischen Salz und der bestimmten Pseudozufallszahl in wenigstens eine der Hash-Funktionen aufweist, um den jeweiligen Schlüssel zu erzeugen.

10. Verfahren nach einem der vorhergehenden Ansprüche, wobei die ein oder mehr Modifikationsalgorithmen ein oder mehr linguistische Modifikationsalgorithmen und/oder ein oder mehr numerische Modifikationsalgorithmen aufweisen.

11. Verfahren nach einem der vorhergehenden Ansprüche, wobei die ein oder mehr Kennungen wenigstens eines aufweisen von: einem Vornamen, einem Nachnamen, einem Geburtsdatum, einer Staatsangehörigkeit, einer Geburtsstadt, einer Adresse, einer Passnummer, einer Sozialversicherungsnummer, einer Führerscheinnummer, einer Fahrzeugregistrierungsnummer, einer Firmenregistrierungsnummer, einer Vertragsnummer, einer Internetprotokolladresse und/oder einer biometrischen Kennung.

12. Verfahren nach einem der vorhergehenden Ansprüche, wobei das Anwenden ein Anwenden von mehreren Modifikationsalgorithmen auf die wenigstens eine der Kennungen des Datensubjekts entsprechend einem der Einträge im Datensatz der ersten Partei (102a) aufweist.

13. Verfahren zum Vergleichen von Datensatzeinträgen von zwei oder mehr Parteien (102), wobei jede Partei (102) einen Datensatz mit mehreren Einträgen führt, wobei jeder Eintrag ein entsprechendes Datensubjekt (104) darstellt und ein oder mehr Kennungen dieses Datensubjekts (104) aufweist, wobei das Verfahren ein Betreiben einer Computerausrüstung eines Drittpartei-Übereinstimmungsdienstes (112) anders als die zwei oder mehr Parteien aufweist, um Operationen durchzuführen von:

Bereitstellen von ein oder mehr gemeinsamen Modifikationsalgorithmen für jede der zwei oder mehr Parteien (102), wobei jeder Modifikationsalgorithmus die Kennung modifiziert, auf die sie angewendet wird, um dadurch einen entsprechenden Datenwert zu erzeugen;

Bereitstellen von ein oder mehr gemeinsamen Hash-Funktionen für jede der zwei oder mehr Parteien (102), wobei jede gemeinsame Hash-Funktion, wenn sie auf den entsprechenden Datenwert angewendet wird, einen entsprechenden Schlüssel erzeugt, wobei das Bereitstellen der gemeinsamen Hash-Funktionen aufweist:

Bereitstellen einer ersten gemeinsamen Hash-Funktion für jede der zwei oder mehr Parteien (102), wobei die erste gemeinsame Hash-Funktion, wenn sie auf den entsprechenden Datenwert angewendet wird, einen entsprechenden privaten Schlüssel erzeugt; und

Bereitstellen einer zweiten gemeinsamen Hash-Funktion für jede der zwei oder mehr Parteien (102), wobei die zweite gemeinsame Hash-Funktion, wenn sie auf ein oder mehr der privaten Schlüssel angewendet wird, die von jeder Partei erzeugt werden, einen entsprechenden öffentlichen Schlüssel erzeugt, und

wobei das Verfahren ferner aufweist:

Bereitstellen eines gemeinsamen Verschränkungsalgorithmus für jede der zwei oder mehr Parteien (102), wobei der Verschränkungsalgorithmus vorschreibt, welche der erzeugten privaten Schlüssel und/oder welche Teile der erzeugten privaten Schlüssel in die zweite gemeinsame Hash-Funktion eingegeben werden;

Empfangen eines ersten Schlüsselsatzes von einer ersten der zwei oder mehr Parteien, wobei der erste Schlüsselsatz von der ersten Partei (102a) erzeugte Schlüssel aufweist;

Empfangen eines zwei Schlüsselsatzes von einer zweiten der zwei oder mehr Parteien, wobei die erste Partei (102a) und die zweite Partei (102b) unterschiedliche der zwei oder mehr Parteien sind, wobei der zweite Schlüsselsatz von der zweiten Partei (102b) erzeugte Schlüssel aufweist;

Veröffentlichen des ersten und/oder zweiten Schlüsselsatzes an ein oder mehr der zwei oder mehr Parteien (102);

Vergleichen von ein oder mehr der Schlüssel des ersten Schlüsselsatzes mit ein oder mehr der Schlüssel des zweiten Schlüsselsatzes;

Bestimmen, ob das eine der Datensubjekte einem Eintrag in sowohl dem Datensatz der ersten Partei (102a) als auch dem Datensatz der zweiten Partei (102b) entspricht, basierend darauf, ob die verglichenen Schlüsselsätze ein oder mehr identische entsprechende Schlüssel aufweisen; und

Übertragen eines Ergebnisses der Bestimmung an die erste und/oder zweite Partei, wobei das Ergebnis angibt, ob das eine der Datensubjekte einem Eintrag in sowohl dem Datensatz der ersten Partei (102a) als auch dem Datensatz der zweiten Partei (102b) entspricht.

14. Computerprogramm zum Vergleichen von Datensatzeinträgen von zwei oder mehr Parteien (102), wobei jede Partei (102) einen Datensatz mit mehreren Einträgen führt, wobei jeder Eintrag ein entsprechendes Datensubjekt (104) darstellt und ein oder mehr Kennungen dieses Datensubjekts (104) aufweist; wobei das Computerprogramm Anweisungen aufweist, die auf einem computerlesbaren Speicher verkörpert sind und so konfiguriert sind, dass sie, wenn das Programm von einem Computer ausgeführt wird, den Computer Operationen durchführen lassen von:

auf jeden von ein oder mehr der Kennungen des Datensubjekts (104), die einem der Einträge im Datensatz der ersten Partei (102a) entsprechen, Anwenden von ein oder mehr gemeinsamen Modifikationsalgorithmen, wobei die ein oder mehr Modifikationsalgorithmen den zwei oder mehr Parteien gemeinsam sind, wobei jeder Modifikationsalgorithmus die Kennung modifiziert, auf die sie angewendet wird, um dadurch einen entsprechenden Datenwert zu erzeugen;

für jeden erzeugten Datenwert Eingeben wenigstens dieses Datenwerts auf eine gemeinsame Hash-Funktion, die den zwei oder mehr Parteien gemeinsam ist, um einen entsprechenden Schlüssel zu erzeugen, wobei das Erzeugen des entsprechenden Schlüssels aufweist:

für jeden erzeugten Datenwert, Eingeben wenigstens dieses Datenwerts in eine erste gemeinsame Hash-Funktion, um einen entsprechenden privaten Schlüssel zu erzeugen;

Eingeben von ein oder mehr der von der ersten Partei (102a) erzeugten entsprechenden privaten Schlüsseln in eine zweite gemeinsame Hash-Funktion, die den zwei oder mehr Parteien gemeinsam ist, um einen entsprechenden öffentlichen Schlüssel zu erzeugen, wobei die ein oder mehr in die zweite gemeinsame Hash-Funktion eingegebenen entsprechenden privaten Schlüssel basierend auf einem gemeinsamen Verschränkungsalgorithmus ausgewählt werden, der den zwei oder mehr Parteien gemeinsam ist, wobei der gemeinsame Verschränkungsalgorithmus vorschreibt, welche der erzeugten privaten Schlüssel und/oder welche Teile der erzeugten privaten Schlüssel in die zweite gemeinsame Hash-Funktion eingegeben werden;

Speichern jedes erzeugten Schlüssels in einem ersten Schlüsselsatz, wobei das Speichern ein Speichern wenigstens jedes erzeugten öffentlichen Schlüssels im ersten Schlüsselsatz aufweist;

Liefern des ersten Schlüsselsatzes zu einem Vergleichsalgorithmus, der konfiguriert ist, um zu bestimmen, ob das eine der Datensubjekte einem Eintrag in sowohl dem Datensatz der ersten Partei (102a) als auch einem Datensatz einer zweiten Partei (102b) entspricht, wobei die ersten und zweiten Parteien unterschiedliche der zwei oder mehr Parteien sind, durch: (i) Vergleichen von ein oder mehr der Schlüssel im ersten Schlüsselsatz mit ein oder mehr Schlüsseln in einem zweiten von der zweiten Partei erzeugten Schlüsselsatz, und (ii) Bestimmen, ob die verglichenen Schlüsselsätze ein oder mehr identische entsprechende Schlüssel aufweisen, wobei das Vergleichen ein Vergleichen von ein oder mehr der öffentlichen Schlüssel im ersten Schlüsselsatz mit ein oder mehr öffentlichen Schlüsseln im von der zweiten Partei erzeugten zweiten Schlüsselsatz aufweist, und wobei das Bestimmen ein Bestimmen aufweist, ob die verglichenen Schlüsselsätze ein oder mehr identische entsprechende öffentliche Schlüssel aufweisen; und

Empfangen eines Ergebnisses des Vergleichsalgorithmus, wobei das Ergebnis angibt, ob das eine der Datensubjekte einem Eintrag in sowohl dem Datensatz der ersten Partei als auch dem Datensatz der zweiten Partei entspricht.

15. Computerprogramm zum Vergleichen von Datensatzeinträgen von zwei oder mehr Parteien (102), wobei jede Partei (102) einen Datensatz mit mehreren Einträgen führt, wobei jeder Eintrag ein entsprechendes Datensubjekt (104) darstellt und ein oder mehr Kennungen dieses Datensubjekts aufweist, wobei das Computerprogramm Anweisungen aufweist, die auf einem computerlesbaren Speicher verkörpert sind und so konfiguriert sind, dass sie, wenn das Programm von einem Computer eines Drittpartei-Übereinstimmungsdienstes (112) anders als die zwei oder mehr Parteien ausgeführt wird, den Computer Operationen durchführen lassen von:

Bereitstellen von ein oder mehr gemeinsamen Modifikationsalgorithmen für jede der zwei oder mehr Parteien (102), wobei jeder Modifikationsalgorithmus die Kennung modifiziert, auf die sie angewendet wird, um dadurch einen entsprechenden Datenwert zu erzeugen; und

Bereitstellen von ein oder mehr gemeinsamen Hash-Funktionen für jede der zwei oder mehr Parteien (102), wobei jede gemeinsame Hash-Funktion, wenn sie auf den entsprechenden Datenwert angewendet wird, einen entsprechenden Schlüssel erzeugt, wobei das Bereitstellen der gemeinsamen Hash-Funktionen aufweist:

Bereitstellen einer ersten gemeinsamen Hash-Funktion für jede der zwei oder mehr Parteien (102), wobei die erste gemeinsame Hash-Funktion, wenn sie auf den entsprechenden Datenwert angewendet wird, einen entsprechenden privaten Schlüssel erzeugt; und

Bereitstellen einer zweiten gemeinsamen Hash-Funktion für jede der zwei oder mehr Parteien (102), wobei die zweite gemeinsame Hash-Funktion, wenn sie auf ein oder mehr der privaten Schlüssel angewendet wird, die von jeder Partei erzeugt werden, einen entsprechenden öffentlichen Schlüssel erzeugt, und

wobei die Operationen ferner aufweisen:

Bereitstellen eines gemeinsamen Verschränkungsalgorithmus für jede der zwei oder mehr Parteien (102), wobei der Verschränkungsalgorithmus vorschreibt, welche der erzeugten privaten Schlüssel und/oder welche Teile der erzeugten privaten Schlüssel in die zweite gemeinsame Hash-Funktion eingegeben werden;

Empfangen eines ersten Schlüsselsatzes von einer ersten der zwei oder mehr Parteien, wobei der erste Schlüsselsatz von der ersten Partei (102a) erzeugte Schlüssel aufweist;

Empfangen eines zweiten Schlüsselsatzes von einer zweiten der zwei oder mehr Parteien, wobei die erste Partei (102a) und die zweite Partei (102b) unterschiedliche der zwei oder mehr Parteien sind, wobei der zweite Schlüsselsatz von der zweiten Partei (102b) erzeugte Schlüssel aufweist;

Veröffentlichen des ersten und/oder zweiten Schlüsselsatzes an ein oder mehr der zwei oder mehr Parteien (102);

Vergleichen von ein oder mehr der Schlüssel des ersten Schlüsselsatzes mit ein oder mehr der Schlüssel des zweiten Schlüsselsatzes;

Bestimmen, ob das eine der Datensubjekte (104) einem Eintrag in sowohl dem Datensatz der ersten Partei (102a) als auch dem Datensatz der zweiten Partei (102b) entspricht, basierend darauf, ob die verglichenen Schlüsselsätze ein oder mehr identische entsprechende Schlüssel aufweisen; und

Übertragen eines Ergebnisses der Bestimmung an die erste und/oder zweite Partei, wobei das Ergebnis angibt, ob das eine der Datensubjekte einem Eintrag in sowohl dem Datensatz der ersten Partei (102a) als auch dem Datensatz der zweiten Partei (102b) entspricht.

## Revendications

1. Procédé de comparaison d'entrées d'enregistrements de données d'au moins deux parties (102), dans lequel chaque partie (102) conserve un enregistrement de données comprenant une pluralité d'entrées, chaque entrée représentant une personne concernée correspondante (104) et comprenant un ou plusieurs identifiants de cette personne concernée (104), dans lequel le procédé consiste à faire fonctionner un équipement informatique d'une première desdites au moins deux parties pour effectuer des opérations consistant à :

   appliquer à chacun desdits un ou plusieurs identifiants de la personne concernée qui correspond à l'une des entrées dans l'enregistrement de données de la première partie (120a), un ou plusieurs algorithmes de modification communs qui sont communs auxdites au moins deux parties (102), dans lequel chaque algorithme de modification modifie l'identifiant auquel il est appliqué pour ainsi générer une valeur de donnée respective ;

entrer pour chaque valeur de données générée, au moins cette valeur de données dans une fonction de hachage commune qui est commune auxdites au moins deux parties afin de générer une clé respective, dans lequel ladite étape de génération de la clé respective consiste à :

   entrer pour chaque valeur de données générée, au moins cette valeur de données dans une première fonction de hachage commune pour générer une clé privée respective ;
   entrer une ou plusieurs clés privées respectives générées par la première partie (102a) dans une deuxième fonction de hachage commune qui est commune auxdites au moins deux parties afin de générer une clé publique respective, dans lequel lesdites une ou plusieurs clés privées respectives entrées dans la deuxième fonction de hachage commune sont choisies sur la base d'un algorithme d'intrication commun qui est commun auxdites au moins deux parties, dans lequel l'algorithme d'intrication commun prescrit lesquelles des clés privées générées et/ou lesquelles des parties des clés privées générées sont entrées dans la deuxième fonction de hachage commune ;

stocker chaque clé générée dans un premier jeu de clés, dans lequel ladite étape de stockage consiste à stocker au moins chaque clé publique générée dans le premier jeu de clés ;

fournir le premier jeu de clés à un algorithme de comparaison qui est configuré pour déterminer si ladite une des personnes concernées correspond à une entrée à la fois dans l'enregistrement de données de la première partie (102a) et dans un enregistrement de données d'une deuxième partie (102b), dans lequel les première et deuxième parties sont des parties différentes desdites au moins deux parties en : (i) comparant une ou plusieurs des clés dans le premier jeu de clés à une ou plusieurs clés dans un deuxième jeu de clés généré par la deuxième partie, et (ii) déterminant si les jeux de clés comparés comprennent une ou plusieurs clés respectives identiques, dans lequel ladite étape de comparaison consiste à comparer une ou plusieurs des clés publiques dans le premier jeu de clés à une ou plusieurs clés publiques dans le deuxième jeu de clés généré par la deuxième partie (102b), et dans lequel ladite étape de détermination consiste à déterminer si les jeux de clés comparés comprennent une ou plusieurs clés publiques respectives identiques ; et

recevoir un résultat de l'algorithme de comparaison, dans lequel le résultat indique si ladite une des personnes concernées correspond à une entrée à la fois dans l'enregistrement de données de la première partie (102a) et dans l'enregistrement de données de la deuxième partie (102b).

2. Procédé selon la revendication 1, dans lequel le résultat indique une probabilité de savoir si ladite une des personnes concernées correspond à une entrée à la fois dans l'enregistrement de données de la première partie (102a) et dans l'enregistrement de données de la deuxième partie (102b).

3. Procédé selon la revendication 1 ou 2, dans lequel l'algorithme de comparaison est configuré pour déterminer si ladite une des personnes concernées correspond à une entrée à la fois dans l'enregistrement de données de la première partie (102a) et dans l'enregistrement de données de la deuxième partie (102b), en déterminant si les jeux de clés comparés comprennent un nombre de clés identiques supérieur ou égal à un nombre seuil.

4. Procédé selon l'une quelconque des revendications précédentes, dans lequel l'algorithme de comparaison est un algorithme de comparaison interne effectué par l'équipement informatique de la première partie (102a), et dans lequel ladite étape de fourniture consiste à recevoir le deuxième jeu de clés de la part de la deuxième partie (102b) et à fournir le deuxième jeu de clés reçu à l'algorithme de comparaison interne.

5. Procédé selon l'une quelconque des revendications 1 à 4, dans lequel l'algorithme de comparaison est un algorithme de comparaison externe effectué par un équipement informatique d'un service tiers de mise en correspondance (112) qui est externe auxdites au moins deux parties, et dans lequel ladite étape de fourniture consiste à transmettre le premier jeu de clés audit service de mise en correspondance.

6. Procédé selon l'une quelconque des revendications précédentes, consistant à :

recevoir un nombre initial commun qui est commun auxdites au moins deux parties afin de déterminer un nombre pseudo-aléatoire commun, et

dans lequel ladite étape d'entrée consiste à entrer le nombre initial commun dans au moins l'une des fonctions de hachage pour générer la clé respective.

7. Procédé selon la revendication 6, consistant à mettre à jour le nombre initial utilisé pour déterminer le nombre pseudo-aléatoire commun.

8. Procédé selon l'une quelconque des revendications précédentes, consistant à :

combiner pour chacune desdites valeurs de données, cette valeur de données avec un même sel cryptographique respectif qui est commun auxdites au moins deux parties pour cette valeur de données, et

dans lequel ladite étape d'entrée d'au moins la valeur de données respective dans la première fonction de hachage consiste à entrer au moins la valeur de données respective en combinaison avec le sel cryptographique dans la première fonction de hachage pour générer la clé privée.

9. Procédé selon les revendications 6 et 8, dans lequel ladite étape d'entrée consiste à entrer au moins la valeur de données respective en combinaison avec le sel cryptographique et le nombre pseudo-aléatoire déterminé dans au moins l'une des fonctions de hachage pour générer la clé respective.

10. Procédé selon l'une quelconque des revendications précédentes, dans lequel lesdits un ou plusieurs algorithmes de modification comprennent un ou plusieurs algorithmes de modification linguistique et/ou un ou plusieurs algorithmes de modification numérique.

11. Procédé selon l'une quelconque des revendications précédentes, dans lequel lesdits un ou plusieurs identifiants comprennent au moins l'un parmi : un prénom, un nom, une date de naissance, une nationalité, une ville de naissance, une adresse, un numéro de passeport, un numéro d'assurance nationale, un numéro de permis de conduire, un numéro d'immatriculation de véhicule, un numéro d'immatriculation de société, un numéro de contrat, une adresse de protocole Internet et/ou un identifiant biométrique.

12. Procédé selon l'une quelconque des revendications précédentes, dans lequel ladite étape d'application consiste à appliquer une pluralité d'algorithmes de modification audit au moins l'un des identifiants de la personne concernée correspondant à l'une des entrées dans l'enregistrement de données de la première partie (102a).

13. Procédé de comparaison d'entrées d'enregistrements de données d'au moins deux parties (102), dans lequel chaque partie (102) conserve un enregistrement de données comprenant une pluralité d'entrées, chaque entrée représentant une personne concernée correspondante (104) et comprenant un ou plusieurs identifiants de cette personne concernée (104), dans lequel le procédé consiste à faire fonctionner un équipement informatique d'un service tiers de mise en correspondance (112) autre que lesdites au moins deux parties pour effectuer des opérations consistant à :

fournir un ou plusieurs algorithmes de modification communs à chacune desdites au moins deux parties (102), dans lequel chaque algorithme de modification modifie l'identifiant auquel il est appliqué pour ainsi générer une valeur de données respective ;

fournir une ou plusieurs fonctions de hachage communes à chacune desdites au moins deux parties (102), dans lequel chaque fonction de hachage commune, lorsqu'elle est appliquée à la valeur de données respective, génère une clé respective, dans lequel l'étape de fourniture des fonctions de hachage communes consiste à :

fournir une première fonction de hachage commune à chacune desdites au moins deux parties (102), dans lequel la première fonction de hachage commune, lorsqu'elle est appliquée à la valeur de données respective, génère une clé privée respective ; et

fournir une deuxième fonction de hachage commune à chacune desdites au moins deux parties (102), dans lequel la deuxième fonction de hachage commune, lorsqu'elle est appliquée à une ou plusieurs des clés privées générées par chaque partie, génère une clé publique respective, et

dans lequel le procédé consiste en outre à :

fournir un algorithme d'intrication commun à chacune desdites au moins deux parties (102), dans lequel l'algorithme d'intrication prescrit lesquelles des clés privées générées et/ou lesquelles des parties des clés privées générées sont entrées dans la deuxième fonction de hachage commune ;

recevoir un premier jeu de clés de la part d'une première desdites au moins deux parties, dans lequel le premier jeu de clés comprend des clés générées par la première partie (102a) ;

recevoir un deuxième jeu de clés de la part d'une deuxième desdites au moins deux parties, la première partie (102a) et la deuxième partie (102b) étant différentes desdites au moins deux parties, dans lequel le deuxième jeu de clés comprend des clés générées par la deuxième partie (102b) ;

publier le premier et/ou deuxième jeu de clés à destination d'une ou plusieurs desdites au moins deux parties (102) ;

comparer une ou plusieurs des clés du premier jeu de clés à une ou plusieurs des clés du deuxième jeu de clés ;

déterminer si ladite une des personnes concernées correspond à une entrée à la fois dans l'enregistrement de données de la première partie (102a) et dans l'enregistrement de données de la deuxième partie (102b) selon que les jeux de clés comparés comprennent ou non une ou plusieurs clés respectives identiques ; et

transmettre un résultat de ladite étape de détermination à la première et/ou deuxième partie, dans lequel le résultat indique si ladite une des personnes concernées correspond à une entrée à la fois dans l'enregistrement de données de la première partie (102a) et dans l'enregistrement de données de la deuxième partie (102b).

**14.** Programme informatique destiné à comparer des entrées d'enregistrement de données d'au moins deux parties (102), dans lequel chaque partie (102) maintient un enregistrement de données comprenant une pluralité d'entrées, chaque entrée représentant un sujet de données correspondant (104) et comprenant un ou plusieurs identifiants de ce sujet de données (104), dans lequel le programme informatique comprend des instructions incorporées dans un moyen de stockage lisible par ordinateur et configurées, lorsque le programme est exécuté par un ordinateur, de manière à amener l'ordinateur à effectuer des opérations consistant à :

appliquer à chacun d'un ou plusieurs des identifiants de la personne concernée (104) qui correspond à l'une des entrées dans l'enregistrement de données de la première partie (102a), un ou plusieurs algorithmes de modification communs, dans lequel lesdits un ou plusieurs algorithmes de modification sont communs auxdites au moins deux parties, dans lequel chaque algorithme de modification modifie l'identifiant auquel il est appliqué pour générer ainsi une valeur de données respective ;

entrer pour chaque valeur de données générée, au moins cette valeur de données dans une fonction de hachage commune qui est commune auxdites au moins deux parties afin de générer une clé respective, dans lequel ladite étape de génération de la clé respective consiste à :

entrer pour chaque valeur de données générée, au moins cette valeur de données dans une première fonction de hachage commune pour générer une clé privée respective ;

entrer une ou plusieurs clés privées respectives générées par la première partie (102a) dans une deuxième fonction de hachage commune qui est commune auxdites au moins deux parties afin de générer une clé publique respective, dans lequel lesdites une ou plusieurs clés privées respectives entrées dans la deuxième fonction de hachage commune sont choisies sur la base d'un algorithme d'intrication commun qui est commun auxdites au moins deux parties, dans lequel l'algorithme d'intrication commun prescrit lesquelles des clés privées générées et/ou lesquelles des parties des clés privées générées sont entrées dans la deuxième fonction de hachage commune ;

stocker chaque clé générée dans un premier jeu de clés, dans lequel ladite étape de stockage consiste à stocker au moins chaque clé publique générée dans le premier jeu de clés ;

fournir le premier jeu de clés à un algorithme de comparaison qui est configuré pour déterminer si ladite une des personnes concernées correspond à une entrée à la fois dans l'enregistrement de données de la première partie (102a) et dans un enregistrement de données d'une deuxième partie (102b), dans lequel les première et deuxième parties sont des parties différentes desdites au moins deux parties en : (i) comparant une ou plusieurs des clés dans le premier jeu de clés à une ou plusieurs clés dans un deuxième jeu de clés généré par la deuxième partie, et (ii) déterminant si les jeux de clés comparés comprennent une ou plusieurs clés respectives identiques, dans lequel ladite étape de comparaison consiste à comparer une ou plusieurs des clés publiques dans le premier jeu de clés à une ou plusieurs clés publiques dans le deuxième jeu de clés généré par la deuxième partie, et dans lequel ladite étape de détermination consiste à déterminer si les jeux de clés comparés comprennent une ou plusieurs clés publiques respectives identiques ; et

recevoir un résultat de l'algorithme de comparaison, dans lequel le résultat indique si ladite une des personnes concernées correspond à une entrée à la fois dans l'enregistrement de données de la première partie et dans l'enregistrement de données de la deuxième partie.

15. Programme informatique pour comparer des entrées d'enregistrement de données d'au moins deux parties (102), dans lequel chaque partie (102) maintient un enregistrement de données comprenant une pluralité d'entrées, chaque entrée représentant un sujet de données correspondant (104) et comprenant un ou plusieurs identifiants de ce sujet de données, dans lequel le programme informatique comprend des instructions incorporées dans un moyen de stockage lisible par ordinateur et configurées, lorsque le programme est exécuté par un ordinateur d'un service tiers de mise en correspondances (112) autre que lesdites au moins deux parties, de manière à amener l'ordinateur à effectuer des opérations consistant à :

fournir un ou plusieurs algorithmes de modification communs à chacune desdites au moins deux parties (102), dans lequel chaque algorithme de modification modifie l'identifiant auquel il est appliqué pour ainsi générer une valeur de données respective ; et

fournir une ou plusieurs fonctions de hachage communes à chacune desdites au moins deux parties (102), dans lequel chaque fonction de hachage commune, lorsqu'elle est appliquée à la valeur de données respective, génère une clé respective, dans lequel l'étape de fourniture de la fonction de hachage commune consiste à :

fournir une première fonction de hachage commune à chacune desdites au moins deux parties (102), dans lequel la première fonction de hachage commune, lorsqu'elle est appliquée à la valeur de données respective, génère une clé privée respective ; et

fournir une deuxième fonction de hachage commune à chacune desdites au moins deux parties (102), dans lequel la deuxième fonction de hachage commune, lorsqu'elle est appliquée à une ou plusieurs des clés privées générées par chaque partie, génère une clé publique respective, et

dans lequel les opérations consistent en outre à :

fournir un algorithme d'intrication commun à chacune desdites au moins deux parties (102), dans lequel l'algorithme d'intrication prescrit lesquelles des clés privées générées et/ou lesquelles des parties des clés privées générées sont entrées dans la deuxième fonction de hachage commune ;

recevoir un premier jeu de clés de la part d'une première desdites au moins deux parties, dans lequel le premier jeu de clés comprend des clés générées par la première partie (102a) ;

recevoir un deuxième jeu de clés de la part d'une deuxième desdites au moins deux parties, la première partie (102a) et la deuxième partie (102b) étant différentes desdites au moins deux parties, dans lequel le deuxième jeu de clés comprend des clés générées par la deuxième partie (102b) ;

publier le premier et/ou deuxième jeu de clés à destination d'une ou plusieurs desdites au moins deux parties (102) ;

comparer une ou plusieurs des clés du premier jeu de clés à une ou plusieurs des clés du deuxième jeu de clés ;

déterminer si ladite une des personnes concernées (104) correspond à une entrée à la fois dans l'enregistrement de données de la première partie (102a) et dans l'enregistrement de données de la deuxième partie (102b) selon que les jeux de clés comparés comprennent ou non une ou plusieurs clés respectives identiques ; et

transmettre un résultat de ladite étape de détermination à la première et/ou deuxième partie, dans lequel le résultat indique si ladite une des personnes concernées correspond à une entrée à la fois dans l'enregistrement de données de la première partie (102a) et dans l'enregistrement de données de la deuxième partie (102b).

Figure 1

Figure 2

Figure 3

**EP 3 837 801 B1**

**Patent documents cited in the description**

- US 20078137840 A1
- US 2015288665 A1